# EUROPEAN PATENT APPLICATION

(11) **EP 2 392 276 A1**
(43) Date of publication of application: **07.12.2011**
(21) Application number: 10425186.3
(22) Date of filing: 03.06.2010
(51) Int. Cl.: A61B 17/64

(54) **Ankle clamp device to be affixed to an external fixation system**

(71) Applicant: Orthofix S.r.l., 37012 Bussolengo VR (IT)
(72) Inventor: Venturini, Daniele, 37064 Povegliano Veronese (VR) (IT); Bagnasco, Mara, 20134 Milano (IT)
(74) Representative: Botti, Mario

(57) **Abstract**

Ankle clamp device (1) for the external fixation of bone fractures, in particular of ankle fractures, of the type intended for being mounted at the distal end of a single-side external fixation apparatus (2) through a ball joint, the ankle clamp device (1) comprising a stem (20) having a fork end fitted with a pair of tines (23, 24), and a pivoting clamp (25) comprising a first jaw portion (40) and a second jaw portion (50), the pivoting clamp (25) being rotatably mounted between said tines (23, 24) through said first jaw portion (40), said first and second jaw portion (40, 50) comprising a respective shaped seat (44, 45; 54, 55), wherein a respective insert (74, 75, 75', 85) is fixed within each shaped seat of said first and second jaw portion (40, 50) which interposes between the jaw portion receiving it and a rod-shaped screw (9) when the latter, in use, is kept blocked between the first and the second jaw portion, said first and second jaw portion being made of metallic material, whereas said inserts are made of an electrically insulating material.

## Description

### Field of application

The present invention relates to an ankle clamp device to be affixed to the distal end of an external fixation system which is safe in the use of the diagnostic instrument used in the medical field and known as MRI (magnetic resonance imaging),

### Prior art

It is known that it may be hazardous for patients to wear metal devices inside or outside their bodies when they must undergo an examination with MRI device.

In order to protect the patients, special rules have been adopted that require passing some tests, regulated by special standardization boards such as ASTM, for allowing the use of apparatus in patients that undergo the MRI technique.

A series of tests is based on the level of sensitivity to any forces or movements for the device placed in a strong magnetic field.

Another series of tests ensures that there are no problems due to the heating possibly generated by currents induced in placing the device in a floating magnetic field and another series of tests assesses the influence of the external body on the resulting image.

In order to obviate the problem of magnetic field sensitivity, it is known to use non-magnetic materials in constructing the fixation devices, so as to not be subject to magnetic forces if placed in a magnetic field.

On the other hand, as regards the heating caused by the passage of currents induced inside the fixation system, the solution normally used is to adopt high resistivity materials, typically insulating materials.

Among the proposed solutions, there is that of using components whereon an oxide film has been laid on the surface in advance,

However, in the presence of high currents generated by the MRI devices, the insulation obtained with an oxide film on the surface is not sufficient to prevent the passage of currents and the consequent undesired heating of the same component.

For example, if an external fixation system with relevant ankle clamp associated thereto is subject to MRI, it is highly likely that a closed electrical circuit sets between foot, bone screws and the clamp,

A typical example of ankle clamp devices of the prior art may be found in EP 1139891 by the same applicant.

The prior art ankle clamps, however, exhibit some drawbacks.

In fact, they require the use of metal materials that cannot be used by the patients if subject to MRI or, in the case of use for MRI, the configuration of all components in plastic material with a consequent degradation of the structural stiffness of the entire system.

Therefore, there exists the need for an ankle clamp to be affixed to an external fixation system that allows a high system stiffness, typical of metal clamps, but which at the same time can be safely used also when the patient wearing it undergoes an MRI, thus avoiding the formation of electrical circuits that lead to heating, with a simple and rational constructive solution.

The object of the present invention consists in satisfying the above need while solving the disadvantages mentioned above with reference to the prior art.

### Summary of the invention

This object is achieved by an ankle clamp device to be affixed to an external fixation system according to claim 1.

The dependent claims describe preferred and particularly advantageous embodiments of the clamp device according to the invention.

Further features and advantages of the invention will appear more clearly from the following description, made by way of an indicative non-limiting example with reference to the annexed drawings, wherein:

### Brief description of the drawings

- figures 1-3 show different views of an ankle clamp device according to the present invention, affixed to a fixation system;
- figure 4 shows a perspective view of a first jaw portion of a pivoting clamp according to the present invention;
- figure 5 shows a cutaway view of a second jaw portion of the pivoting clamp according to the present invention;

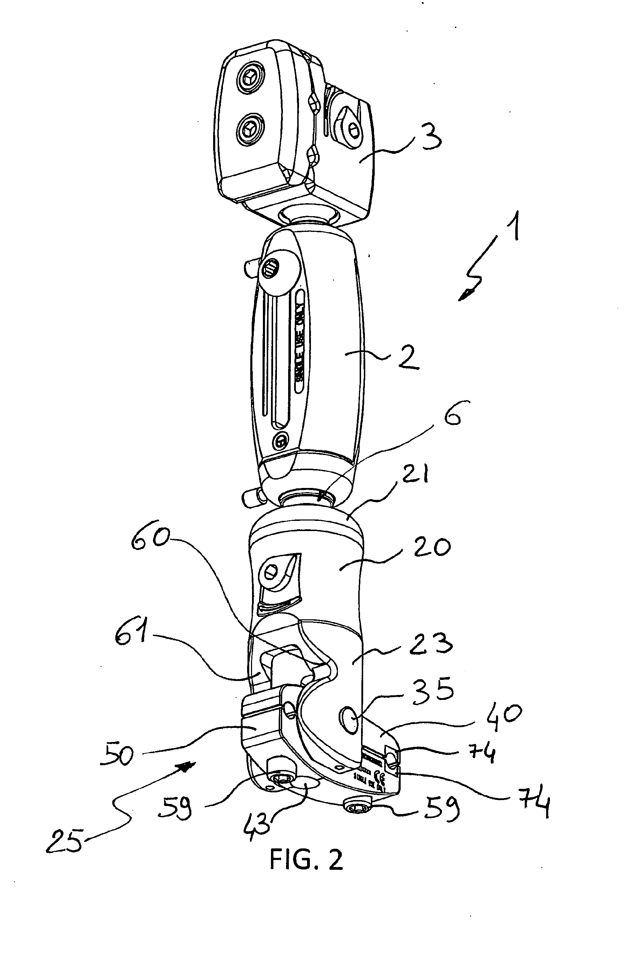

- figure 2 shows a detail of the pivoting clamp according to a first embodiment;

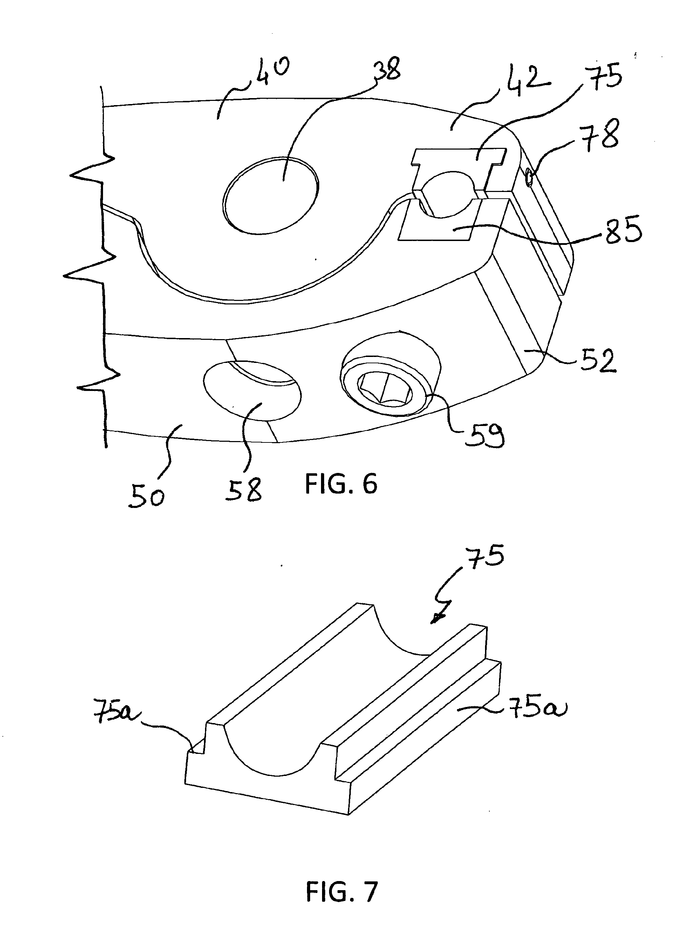

- figure 7 shows an insert element used in the clamp of figure 6;

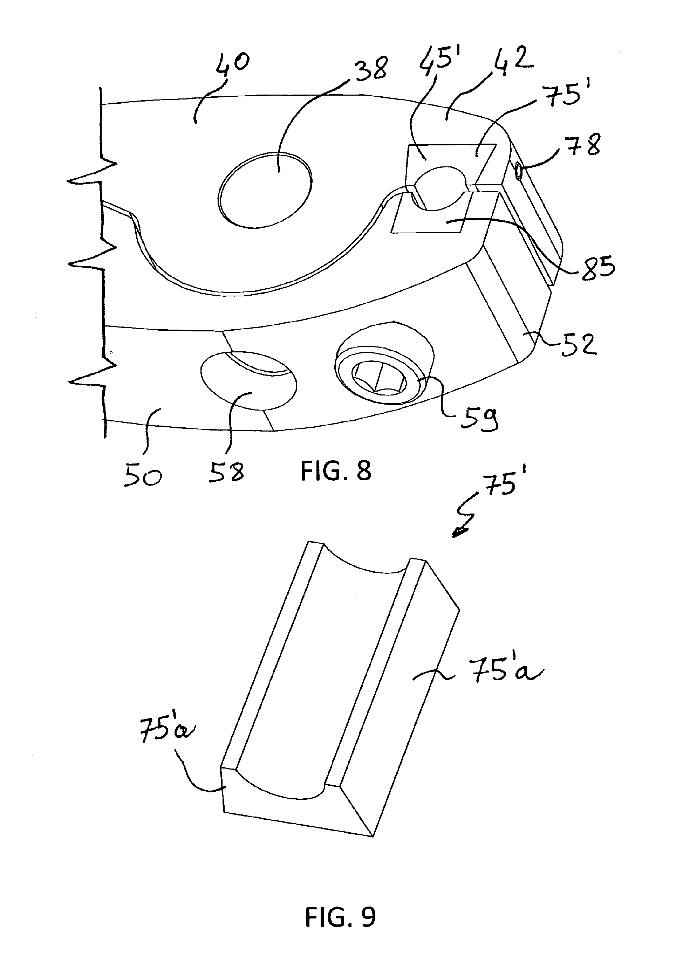

- figure 8 shows a detail of the pivoting clamp according to a second embodiment;
- figure 9 shows an insert element used in the clamp of figure 8;

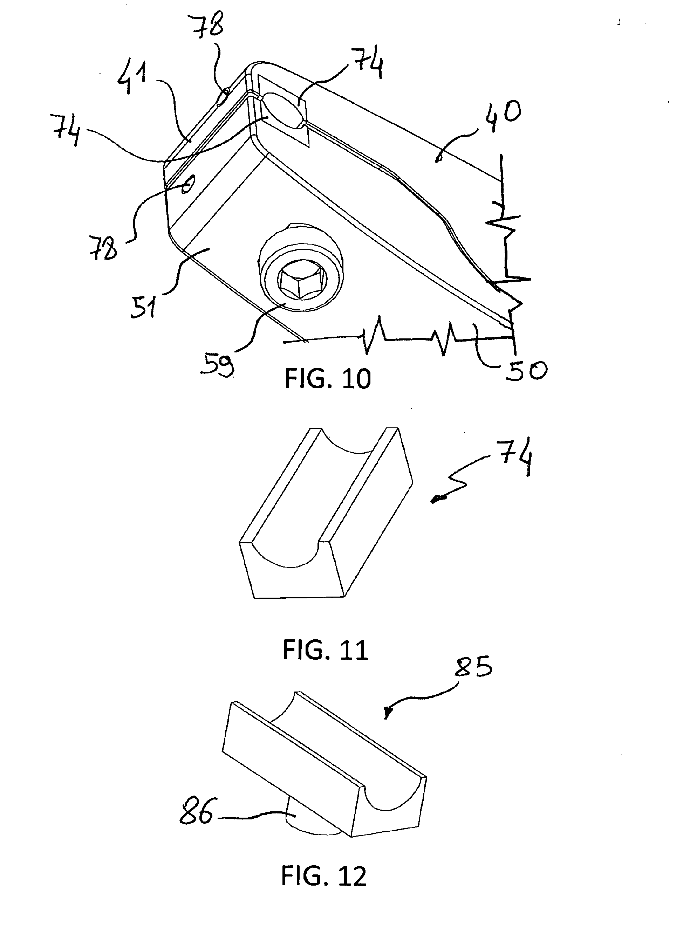

- figure 10 shows a further detail of the pivoting clamp according to the present invention;
- figures 11 and 12 show further insert elements used in the pivoting clamp of figure 10.

### Detailed description

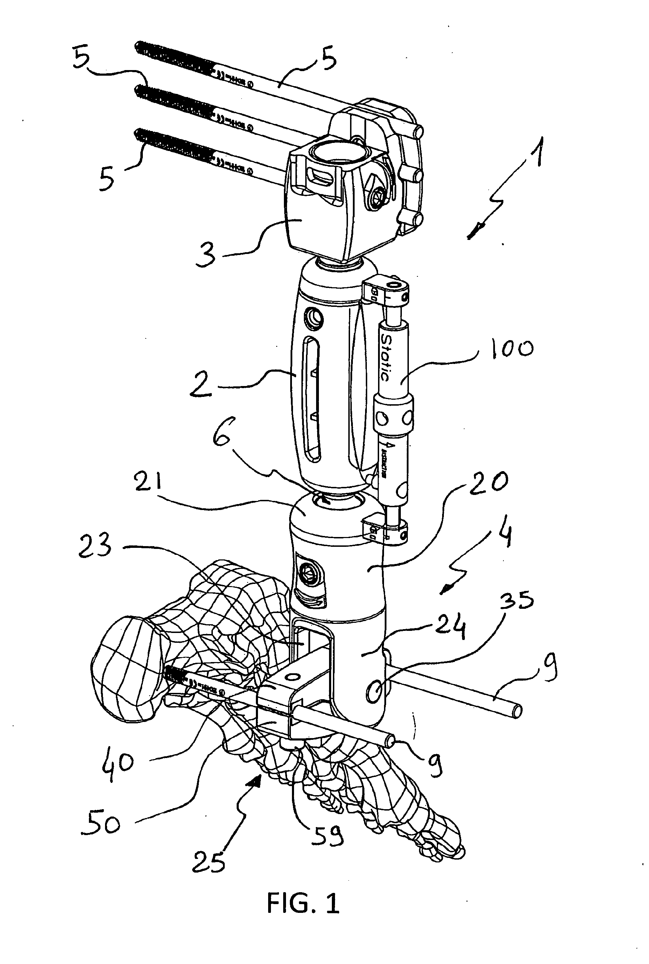

With reference to figures 1-3, reference numeral 1 designates a one-side external fixation apparatus for resolving fractures.

To facilitate the understanding of the methods of application of this fixation apparatus, the perspective view of figure 1 also shows the bone structure of the foot involved in the operation.

Such an apparatus 1 comprises, in its general lines, a central body 2 with an essentially cylindrical shape that can axially be elongated by a distractor 100, the latter being shown in figure 1 only and having opposite ends connected to respective articulated portions.

A first articulated portion, herein referred to as proximal portion 3, is removably constrained to the tibia by some rod-shaped screws 5 inserted in the tibia body. Two or three screws are normally sufficient for ensuring a valid fixation.

The proximal portion 3 is connected to an end of the central body 2 through a ball joint.

A second articulated portion, hereinafter referred to as distal portion 4, is removably constrained to the ankle bones by rod-shaped screws 9 inserted in the talus and in the heel respectively. The distal portion 4 is connected to the other end of the central body 2 through a ball joint 6 with which Allenscrew fixing means are associated.

The distal portion 4 is also called ankle clamp device and in the following description, reference is made to it with this term.

The ankle clamp device 4 according to the present invention, comprises a main body formed by a stem 20, with an essentially cylindrical shape, and a pivoting clamp 25 rotatably mounted at an end of stem 20.

Stem 20 has an end 21 that is connected to the central body of the fixation apparatus and that hereinafter is referred to as top end. The opposite, or bottom end, of stem 20 is shaped as a fork and is provided with a pair of tines 23, 24, between which the pivoting clamp 25 is mounted.

At each of the free ends of tines 23, 24 there is provided a through-hole whose axis lies transverse to the axis Y of stem 20. The holes made in the two ends are coaxial with each other and receive respective opposite ends of a crosswise support pin 35 for the pivoting clamp 25.

Advantageously, according to the present invention, stem 20 is made of a radio-transparent material.

More in particular, stem 20 is preferably made with a fibre-reinforced plastic matrix.

In the embodiment described herein by way of a non-limiting example, a polyetheretherketone or Peek matrix with a 30% with carbon fibres filler is used. The carbon fibres have the purpose to impart a suitable stiffness to the clamp stem.

Of course, other matrices or filler proportions could be used instead.

For example, a carbon fibre of less than 30%, i.e. in the 20% to 30% range, could still provide a sufficient stiffness and enhance the radio-transparency.

Likewise, a fibre fill above 30%, i.e. in the 30% to 50% range, could impart a high stiffness without much impairing the radio-transparency of the stem according to the invention. Moreover, various techniques may be used for making this stem. For example, the stem may be made with a mechanical bar processing, an injection moulding technique or an RTM technique.

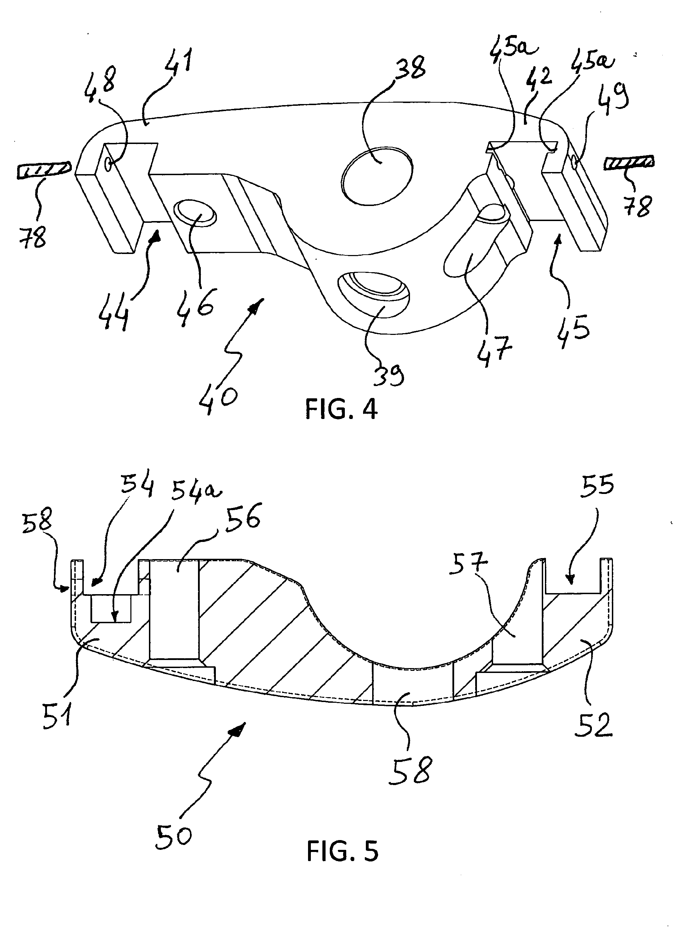

Advantageously, the pivoting clamp 25 comprises a first jaw portion 40 rotatably mounted on pin 35 between tines 23 and 24 of stem 20. Such a first jaw portion 40 comprises a central portion with a rounded profile wherein a through-hole 38 is provided centrally (Fig. 4), Two opposite appendices 41, 42 are formed integral with said central portion.

At each end of appendices 41, 42 there is provided a respective shaped seat 44, 45 intended for accommodating a portion of each of the rod-shaped screws 9 inserted in the talus or heel, in cooperation with a corresponding conjugated seat obtained in a second jaw portion 50 of the pivoting clamp 25 5 described hereinafter.

The axes of the rod-shaped screws 9 accommodated in the two seats 44, 45 are parallel to the axis of hole 38 and lie on a common plane P (Fig: 3). In the proximity of each end of appendices 41, 42 there is formed a respective threaded channel 46, 47 passing through the appendices in a direction perpendicular to the plane P.

A further threaded channel 39 is formed perpendicular to the axis of hole 38 and in communication therewith.

The threaded channel 39 is intended for receiving a fixing screw 43 with Allen head for tightening the first jaw portion 40 of the pivoting clamp 25 in a preferred position defined by the surgeon, wherein the above lying plane P would be inclined at a predetermined angular inclination relative to axis Y of stem 20.

The second jaw portion 50 of clamp 25 has an internal profile conjugated to that of the first jaw portion 40. Also such a second jaw portion 50 comprises appendices 51, 52 at each end whereof there is provided a respective shaped seat 54, 55 intended for cooperating with the corresponding conjugated seat 44, 45 formed in the first jaw portion 40 of the pivoting clamp 25, in order to retain a portion of one of the rod-shaped screws 9 inserted in the talus or in the heel.

The first and the second jaw portion 40 and 50 are joint matching the internal profiles thereof and are made integral to one another thanks to a pair of fixing screws 59 passing through-holes 56, 57 formed in the second jaw portion 50 and collimating with the threaded channels 46, 47 of the first jaw portion 40. Preferably, screws 59 have an Allen head.

A through-hole 58 formed centrally in the second jaw portion 50 further allows screw 43 to reach the threaded channel 39 engaging therein to reach pin 35 against which it abuts for blocking the oscillation of clamp 25.

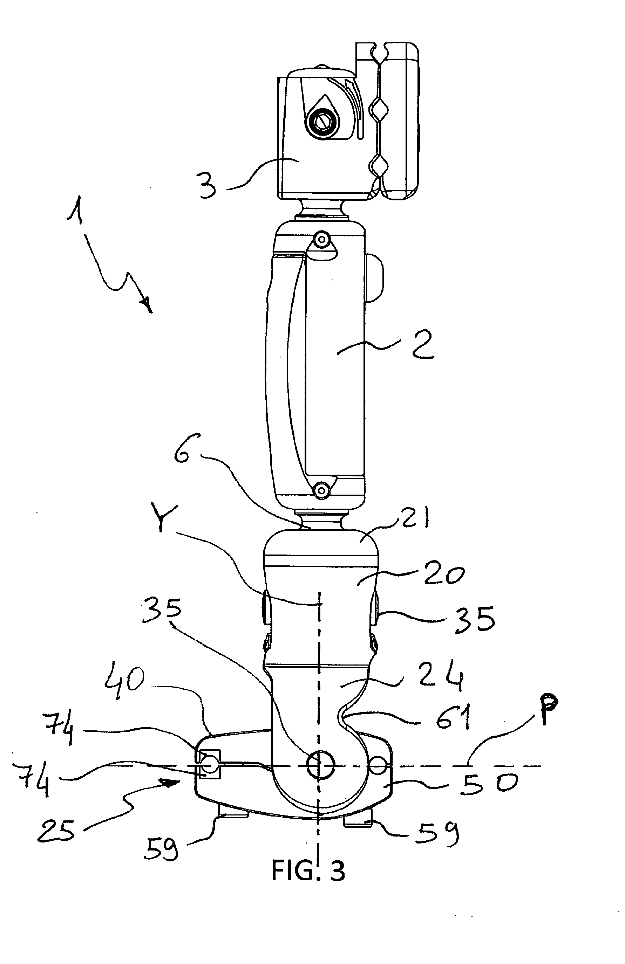

With reference now to axis Y of stem 20 and assuming for pivoting clamp 25 an inclination angle of 0° with respect to the plane P lying perpendicular to this axis Y, as shown in figure 3, an angular travel of the first jaw portion 40 is ensured in a range comprised at least between + 44° and -58°.

Such a wide angular travel range is allowed thanks to the presence of recesses 60 and 61 obtained laterally, and on a single side, in each of tines 23, 24 and in the proximity of their base of attachment to stem 20 (Fig, 2).

The presence of these recesses 60, 61 allows increasing the angular travel range of the pivoting clamp 25 since one of the rod-shaped screws 9 for fixing to the talus or heel, tightened between the first and the second jaw portion 40 and 50 and extending crosswise axis Y of stem 20, may be received in the recesses 60, 61 before abutting against tines 23, 24,

According to the present invention, the first and the second jaw portion 40, 50 are made of a metal material, for example aluminium, so as to impart a high stiffness to the entire system, whereas in order to prevent the creation of closed circuits for the passage of current through the rod-shaped screws 9, the use of suitable inserts 74, 75, 75', 85 is provided, made of an electrically insulating material, interposed between at least one of the rod-shaped screws 9 and the jaw portions 40, 50 in the contact point.

In particular, each insert is individually fixed within the respective seat that receives it. Therefore, there will be a first insert 75 fixed within a first seat 45 of the first jaw portion 40 and a second insert 85 fixed in a second seat 55 of the second jaw portion 50 (Fig. 6).

Fixing the insert into the corresponding seat of the jaw portion is made so as to ensure the stability of the same on the jaw portion, minimising the loss of stiffness of the clamp due to the insulating material the insert is made of.

According to a first embodiment, insert 74 is fit within seat 44, 54 and held fixed into position by a fixing pin or a tightening dowel 78, the end whereof is fixed within insert 74 after having crossed a through-hole 48, 58 formed on the side of appendix 41, 51 of the jaw portion 40, 50 concerned.

The through-hole 48, 58 may be smooth or at least partly threaded.

In addition or as an alternative to the fixing by tightening dowel 78 or pin, the insert, in figure 12 designated with reference numeral 85, may exhibit a central appendix 86 suitable for inserting within a corresponding recess 54a obtained within seat 54 of the jaw portion 50 (Fig. 5).

The tightening dowel 78 exhibits a stem at least partly threaded and at the head top thereof it exhibits a shaped cavity for inserting a screwing tool, such as an Allen wrench. Likewise, the use of a pin, forcedly inserted within the respective hole, may ensure sufficient stability.

According to a second embodiment, seat 45 that receives insert 75 at the bottom exhibits two side recesses 45a (Fig. 4) forming a respective guide wherein two side appendices 75a are inserted that protrude from the base of insert 75 (Fig. 7). This shape forces the side insertion of insert 75 sliding it into guides 45a in the longitudinal direction of the rod-shaped screws 9, when the latter are retained by clamp 25.

Also in this case, the use of a tightening dowel or pin 78 is provided as said above.

According to a third embodiment, seat 45' that receives insert 75' exhibits oblique sides (Fig. 8), forming a trapezoidal profile wherein insert 75' is fitted, also configured with oblique sides 75'a (Fig. 9) so as to fit within seat 45'.

Also in this last-mentioned case, the use of a tightening dowel 78 is provided, fitted laterally in the same way as said above.

The longitudinal extension of each insert is equal to that of the seat that receives it, so as to minimise the portions of plastic material and have the jaw portions of metal material as extended as possible.

Moreover, each insert exhibits a height equal to that of the respective seat of the jaw portion seating it, therefore it does not protrude therefrom.

According to the present invention, all the inserts in any case have a semi-circumferential or V-shaped prism profile on the support side of the rod-shaped screws 9, so as to ensure perfect adherence with the portion of the cylindrical rod-shaped screw that is tightened between the first and the second jaw portion 40, 50.

By way of an indication, in order to make the insert it is possible to use the material known as Peek or Radel, optionally filled with fibres that do not cause the loss of a-magnetic property of the material of the electrical insulation power.

In any case, any other material with high electrical insulation features may be used as an alternative to those just proposed.

The ankle clamp device according to the present invention has the great advantage of ensuring a high structural stiffness, typical of metal clamps, combined with the possibility of allowing the patient wearing it to undergo an examination with an MRI device with no contraindications,

It is clear that a person skilled in the art may make several changes and variations to the ankle clamp device for the external fixation of bone fractures described above in order to meet specific and incidental needs, all falling within the scope of protection of the invention as defined by the following claims.

## Claims

1. Ankle clamp device (1) for the external fixation of bone fractures, in particular of ankle fractures, of the type intended to be affixed to the distal end of a single-side external fixation apparatus (2) through a ball joint, the ankle clamp device (1) comprising a stem (20) having a fork end fitted with a pair of tines (23, 24), and a pivoting clamp (25) comprising a first jaw portion (40) and a second jaw portion (50), the pivoting clamp (25) being rotatably mounted between said tines (23, 24) through said first jaw portion (40), said first and second jaw portion (40, 50) comprising a respective shaped seat (44, 45; 54, 55), **characterised in that** a respective insert (74, 75, 75', 85) is fixed within each shaped seat of said first and second jaw portion (40, 50) which is interposed between the jaw portion receiving it and a rod-shaped screw (9) when the latter, when in use, is kept blocked between the first and the second jaw portion, said first and second jaw portion being made of metallic material, whereas said inserts are made of an electrically insulating material.

2. Device according to claim 1, wherein said first and second jaw portion (40, 50) respectively comprise a central portion and two opposite appendices (41, 42; 51, 52) formed integrally with their central portion, in each of said appendices the shaped seat being obtained so that the pivoting clamp (25) is suitable for seating two-rod shaped screws (9).

3. Device according to claim 2, wherein said insert is only provided in a pair of seats facing each other, respectively a first insert (75, 75') is inserted within a first seat (45) of the first jaw portion (40) and a second insert (85) is inserted within a second seat (55) of the second jaw portion (50).

4. Device according to any one of the previous claims, wherein said insert (85) exhibits a central appendix (86) suitable for inserting within a corresponding recess (54a) formed within the seat (55) wherein it is fixed.

5. Device according to any one of the previous claims, wherein the seat (45) that receives the insert (75) at the bottom exhibits two side recesses (45a) forming a respective guide wherein two side appendices (75a) are inserted that protrude from the base of the insert (75).

6. Device according to any one of the previous claims 1-5, wherein the seat (45') that receives the insert (75') exhibits oblique sides forming a trapezoidal profile wherein the insert (75') is fitted, also configured with oblique sides (75'a).

7. Device according to any one of the previous claims, wherein said insert (74, 75, 75', 85) is fitted into the respective seat (44, 45; 54, 55) and exhibits a longitudinal extension and a height equal to that of the seat it is fixed to.

8. Device according to any one of the previous claims, wherein said insert (74, 75, 75', 85) has a semi-circumferential or V-shaped prism profile on the support side of the rod-shaped screws (9) when the latter are retained by the pivoting clamp (25).

9. Device according to any one of the previous claims, wherein said insert comprises Peek or Radel material.

10. Device according to any one of the previous claims, wherein said insert (74, 75, 75', 85) is held fixed in position by a tightening dowel (78), of which the end is screwed into the insert after having passed through a through-hole (48, 49, 58) located on the jaw portion (40, 50) concerned.

11. Device according to any one of claims 1-9, wherein said insert (74, 75, 75', 85) is held fixed in position by a pin driven into a through-hole (48, 49, 58) located on the jaw portion (40, 50) concerned.
